# EUROPEAN PATENT SPECIFICATION

(11) **EP 3 716 947 B1**
(45) Date of publication and mention of the grant of the patent: **04.12.2024**
(21) Application number: 18822011.5
(22) Date of filing: 30.11.2018
(51) Int. Cl.: A61K 9/06, A61K 47/32, A61K 47/02, A61K 47/26, A61K 31/167, A61K 31/445, A61P 17/02, A61P 23/02

(54) **STABLE WOUND CARE FORMULATION**
STABILE WUNDPFLEGEFORMULIERUNG
FORMULATION STABLE DE SOINS POUR PLAIES

(30) Priority: 30.11.2017 GB 201719982
(43) Date of publication of application: 07.10.2020
(73) Proprietor: Jenarron Therapeutics Limited, Newtownabbey, County Antrim BT36 4NZ (GB)
(72) Inventor: MCCARRON, Paul, Anthony, Newtownabbey County Antrim BT36 4NZ (GB); LOUGHLIN, Ryan, Gerald, Banbridge County Down BT32 4RW (GB)
(74) Representative: MacLachlan & Donaldson
(86) International application number: PCT/EP2018/083164
(87) International publication number: WO 2019/106154

(56) References cited:
- WO-A1-2016/123693
- JP-A- H04 222 892
- US-A1- 2010 040 538
- US-A1- 2010 055 153
- US-A1- 2010 286 205
- LOUGHLIN R G ET AL: "Modulation of gel formation and drug-release characteristics of lidocaine-loaded poly(vinyl alcohol)-tetraborate hydrogel systems using scavenger polyol sugars", EUROPEAN JOURNAL OF PHARMACEUTICS AND BIOPHARMACEUTICS, ELSEVIER SCIENCE PUBLISHERS B.V., AMSTERDAM, NL, vol. 69, no. 3, 1 August 2008 (2008-08-01), pages 1135 - 1146, XP023519576, ISSN: 0939-6411, [retrieved on 20080209], DOI: 10.1016/J.EJPB.2008.01.033
- ABDELKADER D H ET AL: "Characterisation and in vitro stability of low-dose, lidocaine-loaded poly(vinyl alcohol)-tetrahydroxyborate hydrogels", INTERNATIONAL JOURNAL OF PHARMACEUTICS, ELSEVIER, NL, vol. 500, no. 1, 21 January 2016 (2016-01-21), pages 326 - 335, XP029416980, ISSN: 0378-5173, DOI: 10.1016/J.IJPHARM.2016.01.046
- DIARMAID J. MURPHY ET AL: "Physical characterisation and component release of poly(vinyl alcohol)-tetrahydroxyborate hydrogels and their applicability as potential topical drug delivery systems", INTERNATIONAL JOURNAL OF PHARMACEUTICS, vol. 423, no. 2, 1 February 2012 (2012-02-01), NL, pages 326 - 334, XP055572428, ISSN: 0378-5173, DOI: 10.1016/j.ijpharm.2011.11.018
- LORA V. ANGELOVA ET AL: "Cosolvent Gel-like Materials from Partially Hydrolyzed Poly(vinyl acetate)s and Borax", LANGMUIR, vol. 27, no. 18, 20 September 2011 (2011-09-20), US, pages 11671 - 11682, XP055304812, ISSN: 0743-7463, DOI: 10.1021/la202179e

## Description

The present invention relates to a stable gel for use in topical treatment of wounds and surface areas of the body. More specifically the invention provides a stable, cavity filling gel formulation with low bioadhesive strength and cohesive integrity which carries at least one pharmaceutical or cosmetically active ingredient.

The invention primarily addresses the area of PVA gels subjected to reversible or partially reversible cross-linking, for use in wound care to deliver one or more substances topically. The substances can be antibiotics or other drugs or anaesthetics or antiseptics.

PVA-Borate hydrogels display unique flow properties that allow for their use in wound treatment. These gels will flow under low shear to fill the wound cavity and can be removed as a solid mass post treatment due to their shear thickening properties. As described in US 2010/286205 A1 and EP 2 203 192 B1, formulating PVA-borate hydrogels with drug substances, such as lidocaine hydrochloride, allows for an ideal platform to deliver drugs to the wound cavity.

It has, however, been found that the PVA-borate hydrogels described above are prone to instability. In particular, the viscosity and the appearance of known PVA-borate hydrogels changes detrimentally on storage. That is, the viscosity decreases and the system can separate into two phases making it unachievable to remove the gel from a wound in one intact unit. Furthermore, the appearance changes such that the gels go from clear to opaque. This instability can render the gels unsuitable for the treatment of wounds because a sufficient shelf life of equal to or greater than two years cannot be achieved.

The present invention is based upon making a semisolid gel delivery system which is mouldable when handled, acts like a viscous liquid when placed in a space where it has room to flow, which is stable for at least two years on storage and which may be sterilised.

Wound infection may be defined as the entry, growth, metabolic activity and resulting pathophysiological effects of a microorganism upon patient tissue. Wound preparations should ideally be sterilised. Infection has been shown to impair wound healing for both acute and chronic wounds. The increasing resistance of wound infections to both systemic and topical antibiotics has made effective treatment more difficult and accordingly, there is interest in the development of new treatment regimens.

It is an aim of the present invention to provide a PVA-borate gel system incorporating an active ingredient such as a local anaesthetic, for example lidocaine hydrochloride, or antibiotics or photosensitising compounds that form part of PACT and are able to photosensitise bacterial cells or other pathogens making them amenable to the photodynamic effect, wherein the gel system has flow properties suitable for filling a wound, is stable at ambient temperature and pressure for at least two years and may be sterilised.

Surprisingly, it has been found that the aforementioned instability can be overcome by forming PVA-Borate hydrogels which contain less than 5% w/w acetic acid using a PVA grade with a molecular weight between 145,000 and 300,000 Daltons and a degree of hydrolysis of between 98 and 100%.

According to a first aspect of the present invention there is provided a sterile gel formulation suitable for use in filling a wound cavity and delivering an active ingredient thereto, the gel further having a pH range of 4.5 to 8.5, preferably a pH range of 6.5 to 7.5, having low bioadhesive strength and cohesive integrity and being formed from a polymer selected from among the group consisting of poly(vinyl) alcohol (PVA) polymer and a PVA-polyvinyl acetate copolymer, a cross-linker able to form associative interactions between and within said polymer, being a salt form of boron that produces borate ions in aqueous solution such as borax, at least one compound which has a beneficial effect as an active ingredient in the wound and at least one modulator, the modulator being a low molecular weight sugar alcohol that is capable of binding borate or PVA in aqueous solution through a mono-diol or di-diol formation and reduces the pH of PVA-borate hydrogels, wherein the polymer has a degree of hydrolysis of between 98 and 100% and a molecular weight of from 145,000 to 300,000 Daltons, wherein the amount of polymer in the gel formulation ranges from 8 to 20% w/w, and wherein the gel is heat and/or gamma sterilised and contains less than 5% w/w acetic acid.

Such gels retain their clear appearance and viscosity and retain their stability for more than two years.

The modulator is a low molecular weight sugar alcohol possessing a plurality of hydroxyl groups able to bind to borate and most preferably the modulator is D-mannitol.

D-mannitol belongs to a group of chemicals described as sugar alcohols. Other sugar alcohols, whilst may not being as affective as D-mannitol, can also be used to produce the same effect as D-mannitol. Additionally, other low-molecular weight molecules containing two hydroxyl groups on neighbouring carbon atoms, in the cis-position, can also be used to bind borate and reduce the pH of PVA-borate hydrogels, but only low molecular weight sugar alcohols are according to the invention. Other sugar alcohols and compounds possessing the characteristics and structural features of two hydroxyl groups, in the cis-position and attached to adjacent or neighbouring carbon atoms so as to allow presentation of two or more hydroxyl functionalities in a conformation recognisable as a cis conformation, include but is not necessarily limited to maltitol, dulcitol, D-sorbitol, xylitol and meso-erythritol.

Whilst PVA is the best candidate polymer, a copolymer of PVA and polyvinyl acetate could also be used.

The amount of polymer in the gel formulation ranges from 8 to 20 %w/w, preferably 10 to 20% w/w, particularly preferably from 11 to 15% w/w, e.g. 12% w/w.

The molecular weight of the polymer used in practice of the invention is from 145,000 to 300,000 Daltons, preferably from 145,000 to 200,000 Daltons, for example 150,000 Daltons, particularly preferably 145,000 Daltons.

The polymer used in practice of the invention has a degree of hydrolysis of between 98 and 100%, preferably 99%.

The amount of borate used in preparing the gel depends on the type of polymer, e.g. PVA, used. Typically from 0.5 to 5% w/w borate could be used. More typically 1.5 to 4% w/w and most preferably from 1.5 to 3% w/w, e.g. 2.5% w/w. In a particularly preferred embodiment, the amount of borate used in preparing the gel is 1.8% w/w.

The amount of modulator added in the preparation of the gel depends on the polymer, e.g. PVA, and borate and the choice of modulator. Typically from 0.1 to 5% w/w could be used. More preferably from 0.5 to 2% w/w. For example, mannitol is a better modulator than glycerol and therefore less mannitol would be required than glycerol. In a particularly preferred embodiment, the gel contains 2.0% w/w D-mannitol.

Gels prepared according to the invention contain less than 5% w/w 6%- acetic acid, for example between 2 and 4% w/w, preferably less than 2% w/w, particularly preferably less than 1% w/w acetic acid. Ideally, the gels are substantially free from acetic acid.

The gel of the invention can be mounted on or incorporate a support such as a mesh or gauze. This may be advantageous to cover a large surface area. Preferably, the support is provided with adhesive, e.g. an adhesive border, for attachment to a patient's skin.

Upon application to intact skin, the gel according to the invention will flow and take on a convex shape. A thin, wedge-shaped peripheral edge is formed that begins to dry from the edge inwards. Once dry, the gel is wrinkled and rough and no longer adheres to the skin.

In a preferred embodiment, the sterile gel formulation is used in combination with a porous support such as paper, mesh or gauze to form a patch. Placing the porous support onto the gel after application of the gel to a surface prevents the radial drying effect described above. The porous support changes the drying profile such that the gel dries downwards perpendicularly to the surface. The porosity of the outer support can be adjusted to modify the drying profile as desired.

Without being bound by theory, it is suggested that when the patch-like gel with porous support is applied to skin, it flows to make intimate contact with the skin and immediately begins to hydrate it. This opens aqueous skin channels allowing the active ingredient in the gel to permeate the skin. Due to the porosity of the outer support, water slowly evaporates from the outer surface. As a result, the height of the patch slowly decreases and the volume of the patch reduces accordingly. It is reasonable to assume that this increases the amount of active ingredient per unit volume. Therefore, over time, the patch self-compensates for the loss of active ingredient into the skin and automatically adjusts the concentration gradient upwards, thus maintaining the driving force of active ingredient into the skin.

In one preferred embodiment the active ingredient is lidocaine, preferably a salt form of lidocaine, most preferably lidocaine hydrochloride monohydrate. Alternatively, the active ingredient can be another amide local anaesthetic such as prilocaine, bupivacaine etc. or indeed any active ingredient that produces a conjugate acid and is stable in the presence of borate ions.

The amount of active ingredient added in the preparation of the gel depends on the choice of active agent. Typically from 0.1 to 10% w/w could be used. More preferably from 0.5 to 5% w/w, e.g. 4% w/w.

The gel formulations described herein are intended for use in the topical treatment of wounds, in particular for use in wound protection, sealing a wound, preventing blood loss from a wound, the prevention of wound infection and/or the removal of debris from a wound. Examples of debris that may be removed from a wound using the gel formulations described herein are grit, dust, glass and loose tissue.

Accordingly, in a further aspect the present invention provides for gel formulations for use in wound protection, sealing a wound, preventing blood loss from a wound, the prevention of wound infection and/or the removal of debris from a wound.

In embodiments not according to the invention, the gel formulations described herein are also intended for use in topical treatment of intact surface areas of the body, in particular for use in the treatment of pain and/or injuries. For example, the gel formulations described herein may be used in the topical treatment of one or more conditions selected from among contusions, Morton's neuroma, sunburn, general neuralgias, soft tissue injuries, e.g. to fingers and toes, sprains, e.g. sprained ankles and wrists, rheumatoid joints, e.g. rheumatoid finer and toe joints, gout, e.g. gout in toes, post herpetic pain, scars, e.g. painful scars, back pain, broken bones, e.g. broken toes, fingers or ribs, scalds, minor burns, nettle stings, insect bites, vasculitis, chilblains, cold sores, corns, bunions and tendonitis, e.g. tendonitis in ankle, elbow, wrist, knee or shoulder.

In a further aspect, the invention provides a process for preparing the sterilised gel formulation of the invention, comprising:
(a) preparing a stock solution of polymer;
(b) preparing a stock solution of cross-linker;
(c) adding an active ingredient and at least one modulator to the cross-linker solution;
(d) gradually adding the solution prepared in step (c) to the polymer solution prepared in step (a) with stirring to form a gel;
(e) maintaining the resultant gel in a water-bath at 85°C for 30 minutes;
(f) thoroughly stirring the gel to make it as homogenous as possible; and
(g) sterilising the gel with heat or with gamma radiation,
   wherein the polymer (and the amount thereof), cross-linker, active ingredient and modulator are as described as hereinbefore.

In a preferred embodiment, heat sterilisation is carried out at a temperature of from 121°C to 131 °C, particularly preferably for a duration of from 5 to 20 minutes, e.g. 121 °C for 15 minutes.

In yet a further aspect, the gel formulation described herein is for use in a method of treatment of a wound, said method comprising at least the following steps:
(a) applying the gel formulation described herein to an open wound and allowing it to flow to fill the wound cavity;
(b) removing the gel formulation from the wound cavity after a predetermined period of time. Generally, the gel formulation will be an intact mass upon removal.

The gel formulation resides within the wound cavity for a predetermined period of time to allow absorption of the active ingredient such that the active ingredient may exert a clinical effect. This predetermined period of time prior to removal will vary depending on the active ingredient and is preferably in the range of from about 1 minute to about 48 hours, particularly preferably from about 10 minutes to about 6 hours, e.g. about 1 hour to about 4 hours. However, this is not considered to be limiting and other time periods are within the scope of the invention.

In a further aspect that is not according to the invention, the gel formulation described herein is for use in a method of treatment of pain in a human or animal patient, said method comprising at least the following steps:
(a) applying the gel formulation described herein to an intact skin surface in the region of the patient's body where the desired therapeutic effect is to be delivered;
(b) overlying the gel formulation with a support as described herein;
(c) removal of the gel formulation from the skin surface after a predetermined period of time.

The gel formulation is left on the skin surface for a predetermined period of time to allow absorption of the active ingredient such that the active ingredient may exert a clinical effect. This predetermined period of time prior to removal will vary depending on the active ingredient and is preferably in the range of from about 1 minute to about 48 hours, particularly preferably from about 10 minutes to about 6 hours, e.g. about 1 hour to about 4 hours. However, this is not considered to be limiting and other time periods are within the scope of the invention.

In a preferred embodiment of the gel formulation for use in a method of treatment of pain, the support attaches securely to the exposed surface of the applied gel formulation. In this embodiment, the support restricts the movement and drying of the underlying gel formulation. For example, the support may be a fibrous cover for attachment to the gel formulation surface.

In an alternative preferred embodiment, the support is provided with adhesive for attachment to surrounding skin to restrict movement and drying of the underlying gel formulation.

The invention will hereinafter be more particularly described with reference to the following Figures, which show by way of example only, particular embodiments of the gel according to the invention.

In the drawings:
Figures 1A, 1B, 1C, 1D, 1E and 1F are photographs which show the appearance after 9 months of various blank PVA-THB hydrogels (i.e. not containing an active agent);
Figure 2 is a graph which shows the effect of storage (0 vs 9 months) on the pH of the blank PVA-THB hydrogels;
Figures 3A, 3B, 3C, 3D, 3E and 3F are graphs which show the results of stress sweep (0.1 to 100 Pa) at different frequency (0.1, 1, 10 Hz) for the fresh (0 month) and 9 month old PVA-THB hydrogels referred to in Figure 2;
Figures 4A, 4B, 4C, 4D, 4E and 4F are graphs which show the effect of storage duration on storage (G') and loss (G") moduli and loss tangent (G"/G) of the blank PVA-tetrahydroxyborate (THB) hydrogels;
Figures 5A, 5B and 5C are combined graphs which respectively show the effect of storage duration on storage (G') moduli, loss (G") moduli and loss tangent (G"/G) of the blank PVA-THB hydrogels;
Figures 6A and 6B are graphs which show storage (G') and loss (G") moduli of various 9 month old blank PVA-THB hydrogels, before and after reheating;
Figures 7A and 7B are graphs which show storage (G') and loss (G") moduli for various lidocaine HCl loaded PVA-THB hydrogels according to the invention;
Figures 8A and 8B are graphs which show the effect of storage on *in vitro* release of lidocaine HCl from the PVA-THB hydrogels according to the invention;
Figures 9A and 9B are graphs which show storage (G') and loss (G") moduli for lidocaine HCl loaded PVA-THB hydrogels according to the invention; and
Figures 10A and 10B are graphs which show the effect of storage on *in vitro* release of lidocaine HCl from other PVA-THB hydrogels according to the invention.

The following description outlines that low degree of hydrolysis PVA blank hydrogels exhibit turbidity, phase separation and an acetic acid-like odour on storage. These unwanted changes cannot be reversed by simple heating and mixing. Higher degree of hydrolysis (99%) PVA blank hydrogels do not exhibit such changes.

For lidocaine HCl loaded PVA-THB hydrogels, hydrogels prepared with polymers having 99% hydrolysis are superior to lower degree of hydrolysis PVA hydrogels in almost all physicochemical properties and lidocaine HCl is generally stable in the higher degree of hydrolysis formulation at 20°C.

The effect of degree of hydrolysis on the stability of both drug loaded and placebo/blank hydrogels is unexpected. This knowledge can be used to successfully manufacture stable PVA-THB hydrogels.

### EXAMPLES

Measurement of stability parameters was conducted for both Lidocaine loaded and blank (placebo) hydrogels.

### REFERENCE EXAMPLE 1: Blank PVA - THB Hydrogels

### Preparation

PVA and borax were obtained from Merck^{®} (Merck Millipore Catalogue Nos. 141350-354, 141356 and 106303, respectively). PVA stock solution and 5% borax solution were prepared in advance of preparing the hydrogels.

20% PVA stock solution sufficient to result in a final PVA concentration of 10% w/w was weighed into an empty container followed by gradual addition with stirring of 5% borax solution sufficient to result in a final borax concentration of 2.5% w/w. The resultant gel was kept in a water-bath at 85°C for 30 minutes. After heating, the gel was stirred thoroughly to make it as homogenous as possible and poured into containers. The compositions of fresh and 9 month old blank PVA-THB hydrogels are displayed in Table 1 below.

The viscosity of the PVA in standard solution as listed under PVA type in Table 1 above (e.g. 4 mPas, 5 mPas, 28 mPas) is proportional to the molecular weight of the PVA chains.

For example, the molecular weight of 28-99 PVA is about 145,000 Daltons.

**Table 1: Batch number, storage duration, PVA type and composition of fresh and 9 month old blank PVA-THB hydrogels.**

| **Batch No.** | **Storage condition** | **PVA Type*** | **PVA (% w/w)** | **Borax (% w/w)** |
|---|---|---|---|---|
| 1 | 9 months | 4-88 | 10 | 2.5 |
| 2 | 9 months | 5-88 | 10 | 2.5 |
| 3 | 9 months | 8-88 | 10 | 2.5 |
| 4 | 9 months | 26-88 | 10 | 2.5 |
| 5 | 9 months | 40-88 | 10 | 2.5 |
| 6 | 9 months | 28-99 | 10 | 2.5 |
| 7 | 0 months | 4-88 | 10 | 2.5 |
| 8 | 0 months | 5-88 | 10 | 2.5 |
| 9 | 0 months | 8-88 | 10 | 2.5 |
| 10 | 0 months | 26-88 | 10 | 2.5 |
| 11 | 0 months | 40-88 | 10 | 2.5 |
| 12 | 0 months | 28-99 | 10 | 2.5 |

| | | | | |
|---|---|---|---|---|
| *viscosity in mPas (40 g/l water) - degree of hydrolysis (as named by Merck^{®}) | | | | |

All 9 month old blank hydrogels prepared with lower degree of hydrolysis (PVA (4-88, 5-88, 8-88, 26-88 and 40-88)) and higher degree of hydrolysis (PVA (28-99)) were observed for colour or phase changes.

### pH and Rheological measurement

The pH, storage (G'), loss (G") moduli and loss tangent of fresh and 9 month old blank PVA-THB hydrogels (Table 1) were determined. Measurements were carried out using a controlled stress rheometer (AR 1500, TA Instruments, New Castle, DE, USA) with Rheology Advantage Data Analysis Program Version 5.7.0, TA. Parallel plate geometry (20 mm, 1 mm gap) was used for the prepared hydrogels. The experimental temperature was efficiently monitored at 25°C (±0.1°C). The rheometer instrument was programmed for set temperature (25°C) and equilibration for 3 min. Stress sweep (0.1 to 100 Pa) was performed on all samples at three different frequencies (0.1, 1, 10 Hz) to check the linearity of stress vs. strain curve within the range studies. Frequency sweep from 0.1 to 100 Hz at constant stress of 60 Pa was also performed on each samples to find out storage (G') and loss (G") moduli.

### Re-heating Experiments and Effect of Heating Time

Some of the 9 month old hydrogels showed a turbid/opaque appearance and even de-mixing (phase separation). To check the reversibility of such changes, these 9 month old hydrogels were again heated at 85°C for 30 minutes. Various physicochemical parameters such as storage (G'), loss (G") moduli as well as hardness and compressibility were determined for the re-heated 9 month old hydrogels.

To investigate a previously observed phase change on extended heating for 88% hydrolysed PVA, 26-88 and 28-99 hydrogels were freshly prepared with different periods of heating at 85°C (1, 2 and 3 hours). The physical form (homogenous or de-mixed) was observed after cooling to room temperature.

### Results

### Visual Observation

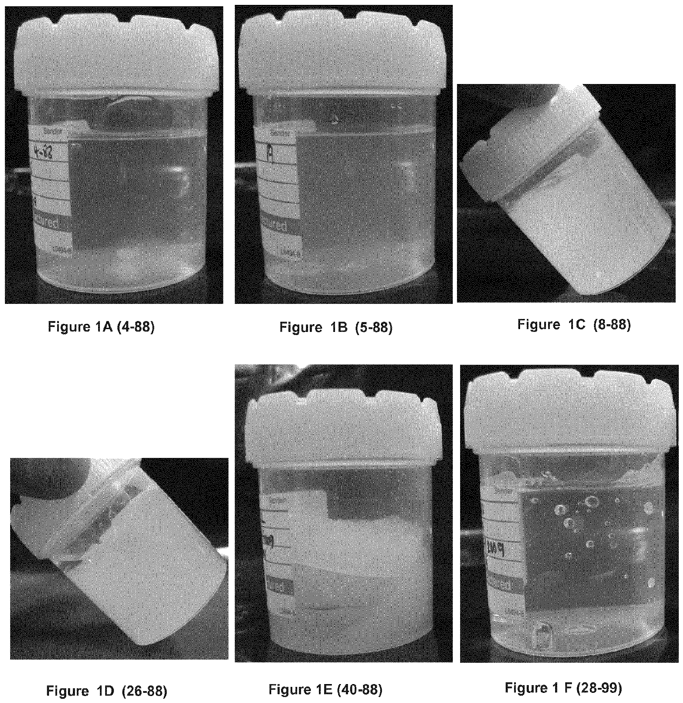

All 9 month old blank hydrogels prepared were observed for physical appearance and phase separation. The representative photographs of PVA blank hydrogels listed in Table 1 (batch numbers 1-6) are shown in Figures 1A-1F, respectively. Containers containing low degree of hydrolysis hydrogels were found to give off an acetic acid like odour. The odour was proportional to the degree of turbidity/phase separation. The above observations are summarised in Table 2 below.

As can be seen in Table 2 and Figure 1, the higher degree of hydrolysis (99%) PVA (28-99) hydrogels remained clear without phase separation. These same hydrogels did not give off an acetic acid like odour. In contrast, the lower degree of hydrolysis (88%) PVA (4-88, 5-88, 8-88, 26-88 and 40-88) hydrogels showed turbidity and phase separation.

The turbidity was proportional to the molecular weight of PVA chains. 4-88, 5-88 and 8-88 hydrogels did not show phase separation but 26-88 and 40-88 hydrogels showed de-mixed phases. De-mixing was more significant with 40-88 hydrogels than with 26-88 hydrogels.

The major difference between the gels which displayed some sort of time dependent change and those which did not is the degree of hydrolysis. This suggests that the observations are attributable to the difference in the number of residual acetate groups present on the PVA chains.

Without being bound by theory, it is suggested that hydrolysis of residual acetate groups leads to the liberation of residual acetic acid groups which causes a reduction in the local pH which discourages the diol-THB interaction and encourages degradation of PVA chains. Furthermore, the resultant degraded PVA chains and disrupted interaction between the diol-groups and THB anions are thought to encourage hydrophobic interactions between nonpolar regions of the polymer which could possibly be sufficient to cause the turbidity seen.

**Table 2: Batch number, colour, phase condition and acetic acid like odour for the 9 month old blank PVA-THB hydrogels prepared according to Table 1.**

| **Batch No.** | **Colour** | **De-mixing** | **Acetic acid like odour** |
|---|---|---|---|
| 1 | Opaque (+) | No | Yes (+) |
| 2 | Turbid (++) | No | Yes (++) |
| 3 | Turbid (+++) | No | Yes (+++) |
| 4 | Turbid (++++) | Yes | Yes (++++) |
| 5 | Turbid (+++++) | Yes | Yes (+++++) |
| 6 | Clear (-) | No | No (-) |

| | | | |
|---|---|---|---|
| Note: '-' = absence of colour or odour; `+' = lowest colour or odour; '+++++' = highest colour or odour. | | | |

### pH

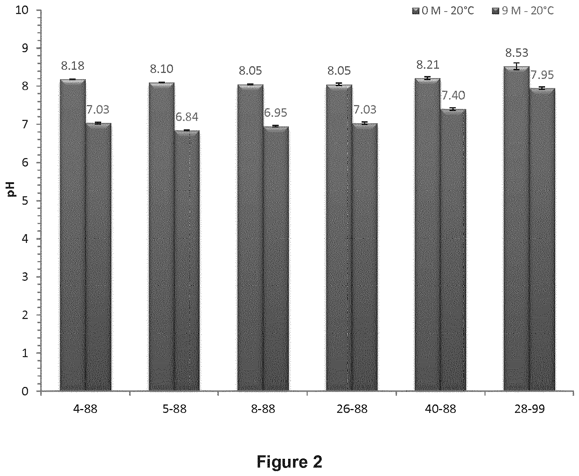

The pH of fresh and 9 month old hydrogels are displayed in Figure 2 with the left hand column in each group of two columns showing the pH of the fresh hydrogel at 20 °C and the right hand columns showing the pH of the 9 month old hydrogel at 20 °C. The decrease in pH is deemed to be related to the degree of hydrolysis and the molecular weight of the PVA used to prepare the gels. As can be seen from Figure 2, the 28-99 PVA hydrogels showed the smallest decrease in pH over 9 months storage at 20°C. Again, without being bound by theory, it is suggested that the hydrolysis of residual acetate groups results in formation of acetic acid over a period of time and hence a decrease in pH.

### Rheological Measurement

Stress sweep and frequency sweep were performed on blank PVA hydrogels. The main objective of stress sweep was to check the linearity of strain vs. stress curves at different frequencies (0.1 to 10 Hz used during frequency sweep). By checking the linearity over the studied frequencies it can be ascertained that the frequency measurement is carried out within the elastic limit.

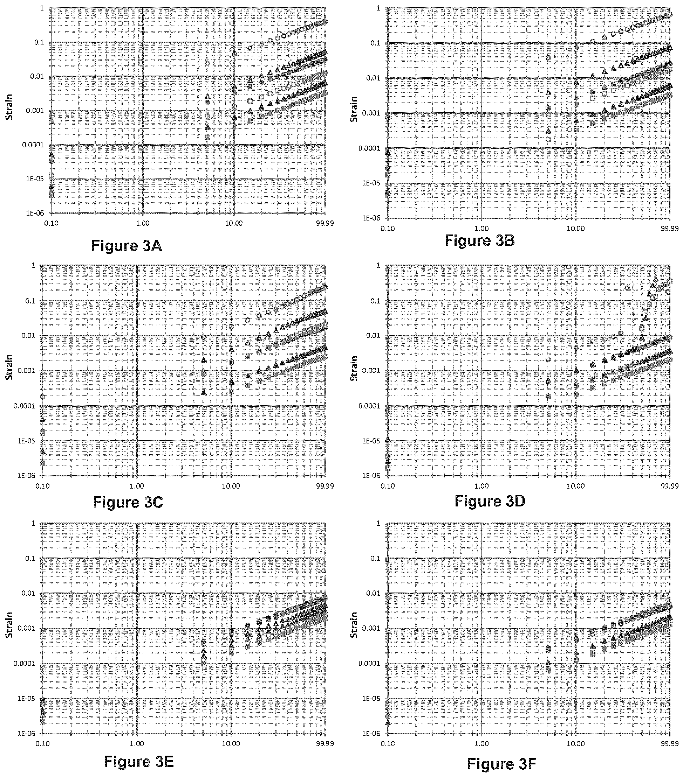

The results of the stress sweep over a range of 0.1 to 100 Pa at three different frequencies (0.1, 1, 10 Hz) are graphically represented in Figures 3A-F, wherein a solid circle represents stress sweep for PVA hydrogel batch numbers 7, 8, 9, 10, 11 and 12, respectively, at 0.1 Hz, a solid triangle at 1 Hz and a solid square at 10 Hz, an empty circle represents stress sweep for PVA hydrogel batch numbers 1, 2, 3, 4, 13 and 6, respectively, at 1.0 Hz, an empty triangle at 1 Hz and an empty square at 10 Hz. All types of PVA-THB hydrogels showed linear strain vs. stress curve over the range of stress and frequency studied except 26-88 blank hydrogels which were 9 months old. These gels were opaque with phase separation giving rise to a turbid polymeric mass with a clear liquid on top.

For gels made with a lower degree of hydrolysis PVA, as the molecular weight (and hence the viscosity) of the PVA increased, the loss of strain after storage for 9 month tended to decrease. The loss of strain after 9 month storage was greatest for 4-88 and least for 40-88 PVA hydrogel. However, the hydrogels were still elastic (linear over the range of stress and frequency studied) after 9 month storage at 20°C, the loss of strain over a period of time indicates a decrease in elasticity of the hydrogels.

As for the higher degree of hydrolysis PVA hydrogel (i.e. 28-99, Figure 3F), the loss of strain after 9 month storage at 20°C was negligible or the strain was the same as the initial values. This result supports the superiority of 28-99 hydrogels over lower degree of hydrolysis PVA hydrogels.

### Frequency sweep

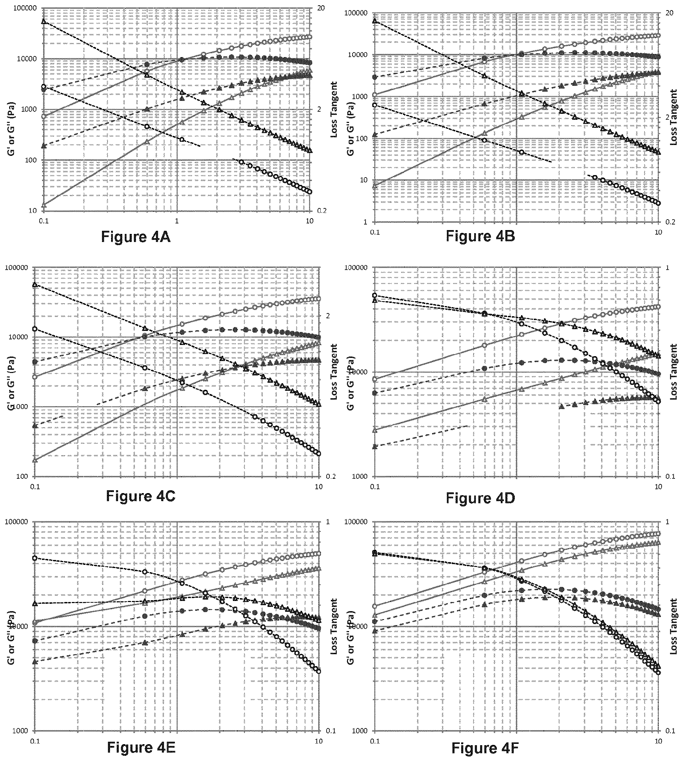

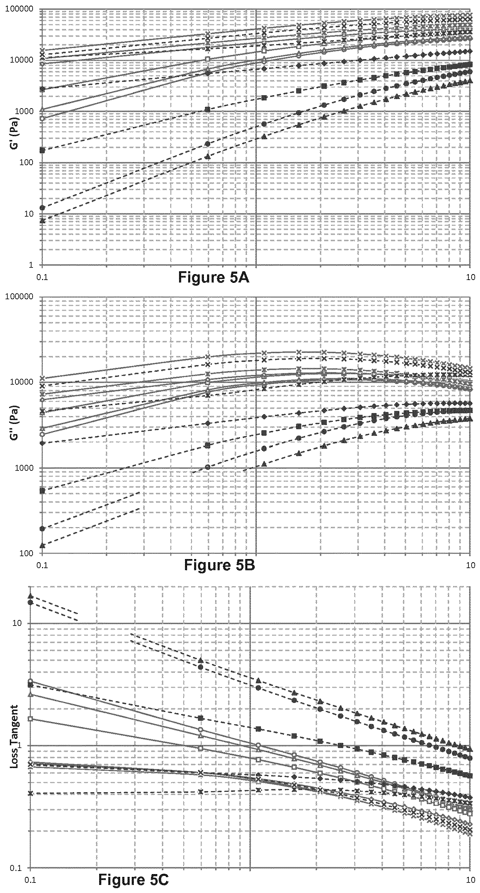

The effects of 9 month storage at 20°C on storage (G'), loss (G") moduli and loss tangent (G"/G') for different PVA type blank hydrogels have been depicted in the individual graphs in Figures 4A-F and the combined graphs in Figures 5A-C, wherein in Figures 4A-F an empty circle with a solid line represents storage (G') moduli, a solid circle with a dotted line loss (G") moduli and an empty circle with a dotted line loss tangent for fresh PVA hydrogel batch numbers 7, 8, 9, 10, 11 and 12 at 20 °C, respectively; an empty triangle with a solid line represents storage (G') moduli, a solid triangle with a dotted line loss (G") moduli and an empty triangle with a dotted line loss tangent for 9 month old PVA hydrogel batch numbers 1, 2, 3, 4, 13 and 6 at 20 °C, respectively.

In the combined graphs shown in Figures 5A-C, storage (G') moduli (Figure 5A), loss (G") moduli (Figure 5B) and loss tangent (Figure 5C) are represented by an empty circle with a solid line for batch number 7, an empty square with a solid line for batch number 9, a cross on a solid line for batch number 11, a solid square on a dotted line for batch number 1, a solid square on a dotted line for batch number 3 and a cross on a dotted line for batch number 13.

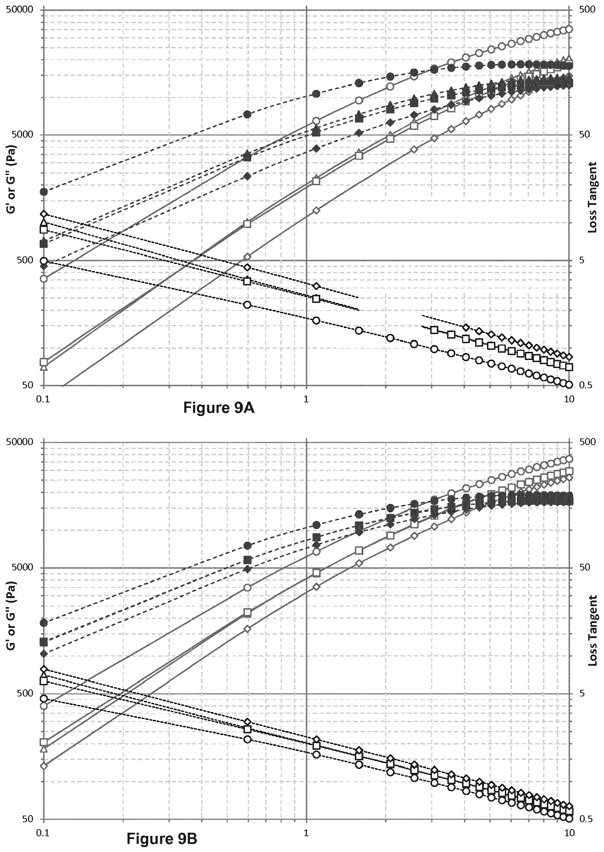

As seen from Figures 4 and 5, 4-88, 5-88, 8-88 and 26-88 PVA hydrogels showed considerable loss of storage and loss moduli. Furthermore, loss of elasticity of the hydrogels is indicated. Similarly, 40-88 PVA hydrogel also showed decrease in such property. However, the magnitude of change was less than for the 4-88, 5-88, 8-88 and 26-88 PVA hydrogels.

Figures 4F and Figures 5A-C show that there was negligible change in storage and loss moduli for 28-99 PVA hydrogels. The higher stability of 28-99 gels could be due to a higher degree of hydrolysis eliminating the possibility of acetic acid formation as explained. Finally, frequency sweep experiments also proved superiority of 28-99 hydrogels over lower degree of hydrolysis PVA hydrogels.

### Re-heating Experiments and Effect of Heating Time

To check whether the turbidity, pH drop and phase separation is reversible or not, 9 month old hydrogels were again re-heated to 85°C for 30 minutes, cooled and observed for all above parameters. In addition, storage (G') and loss (G") moduli as well as hardness and compressibility were measured for re-heated hydrogels.

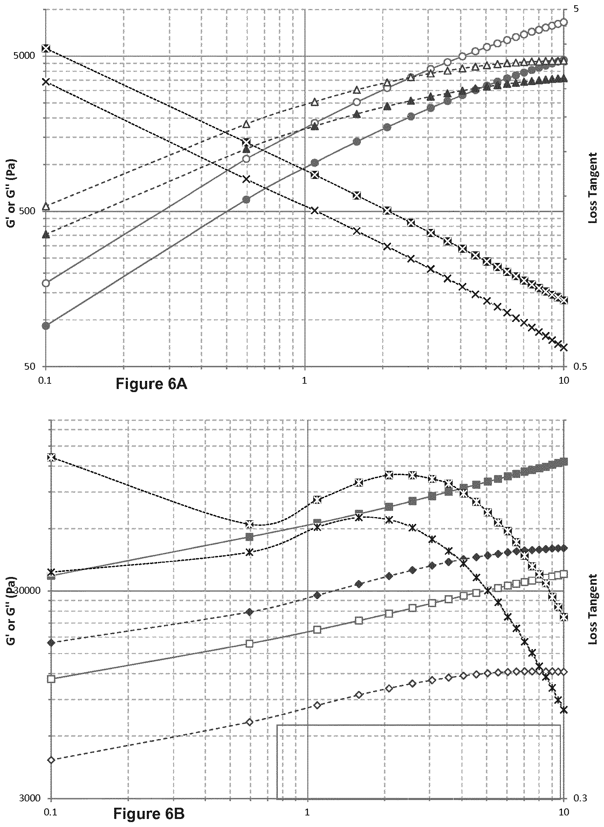

In Figure 6A, storage (G') and loss (G") moduli and loss tangents for 9 month old PVA hydrogels (batch numbers 3 and 13) are respectively represented by an empty circle, empty triangle and cross prior to heating and a solid circle, solid triangle and white on black cross after heating.

As can be seen in Figure 6 and in Table 3 below, after re-heating the hydrogels, turbidity, phase separation, acetic acid-like odour, hardness and compressibility all increased. The phase separation cannot be reversed by reheating. Storage (G') and loss (G") moduli for 8-88 hydrogels slightly decreased (by 101 fold). However, G' and G" increased 105 fold for 40-88 hydrogels after re-heating. Loss tangent increased for both hydrogels 101 and 105 fold for 8-88 and 40-88 hydrogels, respectively. This suggests that re-heating caused the hydrogels to become more solid. This is even more evident from increase in hardness and compressibility values of re-heated hydrogels. The increase in hardness and compressibility was much higher for 40-88 hydrogels compared to 8-88 hydrogels.

All of the above observations suggest that the temperature is a major factor for the stability of the low degree of hydrolysis (88%) PVA hydrogels. The instability (in terms of colour, pH drop, acetic acid-like odour generation) caused by temperature is not reversible through reheating. A possible reason for such behaviour could be that the presence of acetic acid may accelerate PVA degradation.

To further investigate these findings, additional blank hydrogels were prepared as per the composition displayed in Table 4 below with different heating times at 85°C. Two PVA hydrogels were prepared (26-88 and 28-99) by heating at 85°C for 1, 2 and 3 hr.

Table 4 below shows that higher degree of hydrolysis (99%) PVA hydrogels (28-99) are stable without any turbidity, acetic acid-like odour and phase separation even after 3 hr heating. However, the lower degree of hydrolysis (88%) PVA hydrogels (26-88) were not stable after heating for 3 hr at 85°C. They exhibited a slight acetic acid-like odour after 2 hr heating and complete phase separation with strong acetic acid-like odour after 3 hr heating. This set of experiments further confirmed that low degree of hydrolysis (88%) PVA hydrogels (26-88) are susceptible to higher temperature. In addition, all above experiments once again proved superiority of higher degree of hydrolysis (99%) PVA hydrogels as they are stable without noticeable change in physicochemical properties. 28-99 PVA have negligible amounts (<1%) of acetate groups which practically eliminates the possibility of hydrolysis over a period of time. The resultant 28-99 hydrogels are the same as that of fresh hydrogels in various physicochemical properties even after 9 months and did not show any substantial change after heating for 3 hours.

**Table 3: Batch number, PVA type, composition, colour, phase condition and acetic odour on 9 month old blank PVA-THB hydrogels (PVA 10% w/w and Borax 2.5% w/w)**

| **Batch No.** | **Storage duration** | **PVA Type** | **Reheating** | **Colour** | **De-mixing** | **Acetic acid like odour** | **Hardness (N)** | **Compr** essibilit y (N.sec) |
|---|---|---|---|---|---|---|---|---|
| 3 | 9 months | 8-88 | - | Turbid (++) | No (-) | Yes (+) | 0.68±10 | 0.70±0. 04 |
| 3 | 9 months | 8-88 | Yes | Turbid (+++) | Yes (+) | Yes (++) | 0.71±0.03 | 0.72±0. 02 |
| 13 | 9 months | 40-88 | - | Turbid (++++) | Yes (+++) | Yes (+++) | 11.45±1.5 1 | 10.10± 1.64 |
| 13 | 9 months | 40-88 | Yes | Turbid (+++++) | Yes (+++++) | Yes (+++++) | 19.77±2.3 3 | 17.13± 1.54 |

| | | | | | | | | |
|---|---|---|---|---|---|---|---|---|
| Note: '-' = absent; `+' = lowest; '+++++' = highest | | | | | | | | |

**Table 4: Effect of heating time at 85 °C on colour, phase condition and acetic odour on fresh blank PVA-THB hydrogels (PVA 10% w/w and Borax 2.5% w/w)**

| **Batch No.** | **Storage duration** | **PVA Type** | **Heating time** | **Colour** | **De-mixing** | **Acetic acid like odour** |
|---|---|---|---|---|---|---|
| 14 | 0 month | 26-88 | 1h | Clear (-) | No | No (-) |
| 14 | 0 month | 26-88 | 2h | Clear (-) | No | Yes (+) |
| 14 | 0 month | 26-88 | 3h | Turbid (+++) | Yes | Yes (+) |
| 15 | 0 month | 28-99 | 1h | Clear (-) | No | No (-) |
| 15 | 0 month | 28-99 | 2h | Clear (-) | No | No (-) |
| 15 | 0 month | 28-99 | 3h | Clear (-) | No | No (-) |

| | | | | | | |
|---|---|---|---|---|---|---|
| Note: '-' = absent; `+' = lowest; '+++++' = highest. | | | | | | |

### EXAMPLE 2: Lidocaine HCl Loaded PVA - THB Hydrogels (only the hydrogels containing 28-99 PVA are according to the invention)

Stability batches were prepared as per the procedure described in EXAMPLE 1 above and the composition shown in Table 5 below. A brief description of the time points, stability condition and number of containers per condition are given in Table 6 below.

**Table 5: Compositions of lidocaine HCl loaded PVA-THB stability hydrogels**

| **Batch No.** | **PVA Type** | **PVA (% w/w)** | **Borax (% w/w)** | **Mannitol (% w/w)** | **Lidocaine (% w/w)** | **Lidocaine HCl (% w/w)** |
|---|---|---|---|---|---|---|
| 16 | 28-99 | 12 | 2.5 | 2.6 | 4 | 4.93 |
| 17 | 28-99 | 12 | 2.5 | 2.6 | 4 | 4.93 |
| 18 | 4-88 | 17 | 2 | 0.475 | 4 | 4.93 |
| 19 | 4-88 | 17 | 2 | 0.475 | 4 | 4.93 |

**Table 6: Stability plan with stability condition, time points (M = month(s)) and testing performed**

| **Batch No.** | **Stability condition** | **Position** | **Initial** | **1 M** | **2 M** | **3 M** | **6 M** | **Res. 1** | **Total containers / condition** |
|---|---|---|---|---|---|---|---|---|---|
| 16 | 30±2 °C/ 65±5 %RH | upright | 6*†, 1# | 6*, 1# | 6*, 1# | 6*†, 1# | 6*, 1# | 1 | 12 |
| 17 | 20 °C/ RH uncontrolled | upright | 6*†, 1# | 6*, 1# | 6*, 1# | 6*†, 1# | 6*, 1# | 1 | 12 |
| 18 | 30±2 °C /65±5 %RH | upright | 6*†, 1# | 6*, 1# | 6*, 1# | 6*†, 1# | 6*, 1# | 1 | 12 |
| 19 | 20 °C/ RH uncontrolled | upright | 6*†, 1# | 6*, 1# | 6*, 1# | 6*†, 1# | 6*, 1# | 1 | 12 |

| | | | | | | | | | |
|---|---|---|---|---|---|---|---|---|---|
| * Multiple testing carried out on the same sample at different time points # Gel samples tested only once at the time point specified Tests carried out: Weight change *, pH *, Lidocaine Assay #, Rheology #, Hardness and compressibility *, *In vitro* release study † | | | | | | | | | |

The 28-99 PVA hydrogels (batch numbers 16 and 17 in Table 5 above) were evaluated for various parameters up to 6 months storage. The same parameters for batch numbers 18 and 19 (4-88 PVA) up to the 3 month time point are also discussed.

### Batch numbers 16 (28-99, 30°C/65 RH) and 17 (28-99, 20°C uncontrolled RH):

The results of the stability batches are summarised in Table 7. Assay of lidocaine HCl by HPLC showed minor fluctuations (minor increases and decreases) over the 6 months of the study, with no appreciable trends. This indicates that the drug is stable in PVA-THB hydrogels over 6 months without any degradation. Weight loss was observed to increase over a period of time at both storage conditions. However, weight loss is very small and did not substantially affect the final composition of the hydrogels.

Changes in pH were negligible over the study and were typically within the error of the measurement. The hardness and compressibility of batch number 17 (28-99 PVA hydrogels at 20°C uncontrolled RH) remains fairly consistent, in comparison to the 4-88 hydrogels, over the duration of the study.

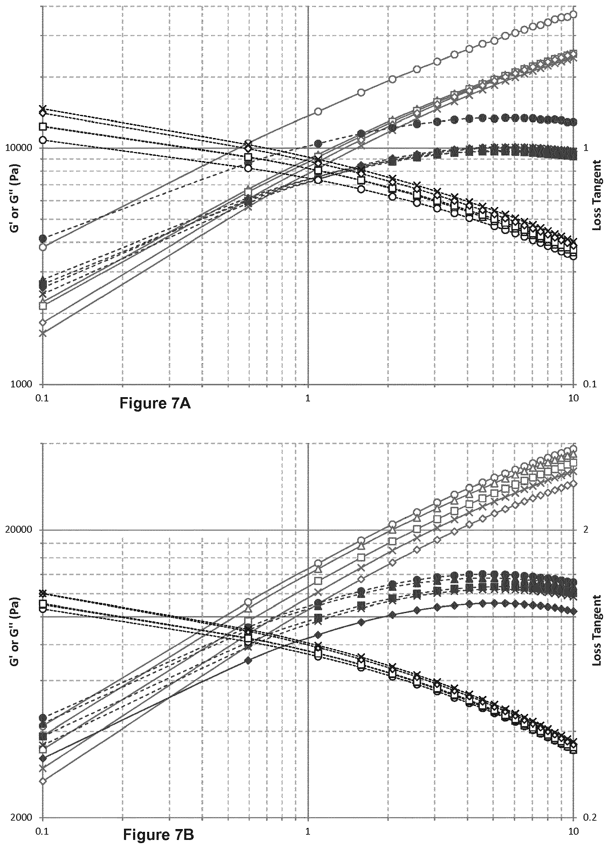

Storage and loss moduli were determined using oscillatory rheometry. A frequency stress sweep was performed at a defined stress of 60 Pa between 0.1 and 10 Hz at 25°C and storage (G') and loss (G") moduli are graphically presented in Figures 7A and 7B for batch numbers 16 and 17 above, wherein a solid line with an empty circle represents the storage (G') moduli for the fresh hydrogel, a solid line with an empty triangle represents the storage (G') moduli for the hydrogel after 1 month, with an empty square two months, empty diamond three months and a cross 6 months, a dotted line with a solid shape the loss (G")moduli, and a dotted line with an empty shape the loss tangent. As can be seen, both moduli decrease considerably at all studied stress frequencies for batch number 16 (30°C/65 RH) over one month storage while remained almost constant during 2, 3 and 6 month storage. Both storage (G') and loss (G") moduli remain fairly constant batch number 17 (20°C/RH uncontrolled) at all storage time points. All of the above observations suggest that ideal storage condition for the gels is 20°C. Storing gels above 20°C cause decrease of storage (G') and loss (G") moduli, hardness and compressibility and pH.

*In vitro* release of lidocaine HCl for batch numbers 16 (30°C/65 RH) and 17 (20°C/RH uncontrolled) after 0, 3 and 6 month storage are graphically depicted in Figures 7A and 7B, respectively. As can be seen from the Figures, the hydrogels were stable and give the same lidocaine HCl release over 30 minutes from different gels within the 6 month period. Neither storage condition (30°C/65 RH and 20°C uncontrolled) nor storage time (0 month to 6 month) yielded any difference in release pattern.

**Table 7: Stability study results for Batch No. 16 (30°C/65 RH) and Batch No. 17 (20°C)**

| | **Parameters** | | | | | | | |
|---|---|---|---|---|---|---|---|---|
| **Stability Condition** | **N ->** | **3** | **12 or 7** | **12 or 7** | **6** | **6** | **6** | **6** |
| | **Time points** | **Lidocain e HCl Assay** | **Weigh t Loss (%)** | **pH** | **Hardnes s (N) at 25°C** | **Com (N.Sec ) at 25°C** | **Hardnes s (N) at 37°C** | **Com (N.Sec ) at 37°C** |
| **30°C/65 RH** Batch No. 16 **28-99 PVA** | 0 M | 104.29 ± 10.07 | - | 7.03 ± 0.02 | 5.90 ± 0.43 | 5.82 ± 0.32 | 1.86 ± 0.13 | 1.76 ± 0.13 |
| | 1 M | 100.30 ± 2.61 | 0.12 ± 0.02 | 6.98 ± 0.02 | 4.53 ± 0.24 | 4.54 ± 0.22 | 1.68 ± 0.11 | 1.58 ± 0.09 |
| | 2M | 96.63 ± 1.59 | 0.21 ± 0.16 | 6.75 ± 0.06 | 4.74 ± 0.38 | 4.56 ± 0.41 | 2.23 ± 0.14 | 2.07 ± 0.15 |
| | 3M | 99.74 ± 3.16 | 0.23 ± 0.17 | 6.81 ± 0.03 | 4.95 ± 0.19 | 4.73 ± 0.26 | 1.67 ± 0.14 | 1.54 ± 0.14 |
| | 6 M | 99.05 ± 1.67 | 0.30 ± 0.17 | 6.88 ± 0.01 | - | - | - | - |
| **20°C** Batch No. 17 **28-99 PVA** | 0 M | 100.88 ± 8.35 | - | 7.00 ± 0.02 | 6.40 ± 0.14 | 6.47 ± 0.14 | 2.21 ± 0.22 | 2.09 ± 0.16 |
| | 1 M | 112.59 ± 9.83 | 0.09 ± 0.03 | 6.99 ± 0.01 | 6.22 ± 0.32 | 6.39 ± 0.30 | 2.33 ± 0.10 | 2.21 ± 0.09 |
| | 2M | 99.95 ± 2.32 | 0.11 ± 0.03 | 6.81 ± 0.02 | 6.49 ± 0.27 | 6.34 ± 0.26 | 2.78 ± 0.30 | 2.65 ± 0.27 |
| | 3M | 102.13 ± 1.48 | 0.12 ± 0.03 | 6.83 ± 0.01 | 6.82 ± 0.24 | 6.70 ± 0.23 | 2.11 ± 0.22 | 1.98 ± 0.16 |
| | 6M | 98.41 ± 2.10 | 0.16 ± 0.03 | 6.92 ±0.02 | 5.47 ± 0.13 | 5.52 ± 0.13 | 2.09 ± 0.16 | 1.96 ± 0.12 |
| **30 °C/65 RH** Batch No. 18 **4-88 PVA** | 0 M | 103.97 ± 1.23 | - | 7.03 ± 0.02 | 3.42 ± 0.21 | 3.21 ± 0.21 | 0.57 ± 0.08 | 0.53 ± 0.09 |
| | 1 M | 105.42 ± 4.32 | 0.09 ± 0.04 | 6.90 ± 0.04 | 2.35 ± 0.21 | 2.18 ± 0.20 | 0.33 ± 0.03 | 0.29 ± 0.03 |
| | 2M | 99.96 ± 0.37 | 0.17 ± 0.05 | 6.87 ± 0.02 | 1.14 ± 0.04 | 1.06 ± 0.05 | 0.25 ± 0.03 | 0.22 ± 0.03 |
| | 3M | 107.55 ± 0.93 | 0.21 ± 0.04 | 6.73 ± 0.02 | 0.95 ± 0.02 | 0.88 ± 0.02 | 0.21 ± 0.02 | 0.20 ± 0.02 |
| | 6 M | - | - | - | - | - | - | - |
| **20°C** Batch No. 19 | 0 M | 100.37 ± 8.45 | - | 7.04 ± 0.02 | 3.08 ± 0.39 | 2.92 ± 0.34 | 0.49 ± 0.04 | 0.46 ± 0.04 |
| **4-88 PVA** | 1 M | 103.89 ± 2.25 | 0.05 ± 0.03 | 6.99 ± 0.02 | 2.89 ± 0.09 | 2.66 ± 0.08 | 0.33 ± 0.03 | 0.30 ± 0.03 |
| | 2 M | 98.54 ± 3.06 | 0.10 ± 0.04 | 6.99 ± 0.03 | 2.81 ± 0.06 | 2.61 ± 0.07 | 0.31 ± 0.03 | 0.28 ± 0.03 |
| | 3 M | 105.76 ± 2.32 | 0.12 ± 0.04 | 6.94 ± 0.01 | 2.52 ± 0.17 | 2.34 ± 0.16 | 0.30 ± 0.03 | 0.27 ± 0.02 |
| | 6 M | - | - | - | - | - | - | - |

### Batch numbers 18 (4-88, 30°C/65 RH) and 19 (4-88, 20°C uncontrolled RH):

The results of the stability batches are summarised in Table 7. The assay of lidocaine HCl by HPLC showed minor fluctuations over the 3 month study. This indicates that the drug is stable in both 28-99 and 4-88 PVA-THB hydrogels up to 3 months without substantial degradation. Weight loss was observed to increase over a period of time at both storage conditions (20°C uncontrolled humidity and 30°C / 65 RH). The weight loss associated with 30°C/65 RH storage condition (Batch number 18) was slightly higher than that associated with 20°C storage condition. However, the weight loss observed was very small and did not substantially affect the final composition of the hydrogels.

The pH was observed to drop over a period of time for both storage conditions. The magnitude of the reduction was greater at 30°C/65 RH compared to 20°C. The hardness and compressibility of 4-88 hydrogels (batch numbers 18 and 19) at both storage conditions continued to drop considerably over the 3 months storage. However, the magnitude of such decrease was lower at 20°C compared to 30°C/65 RH.

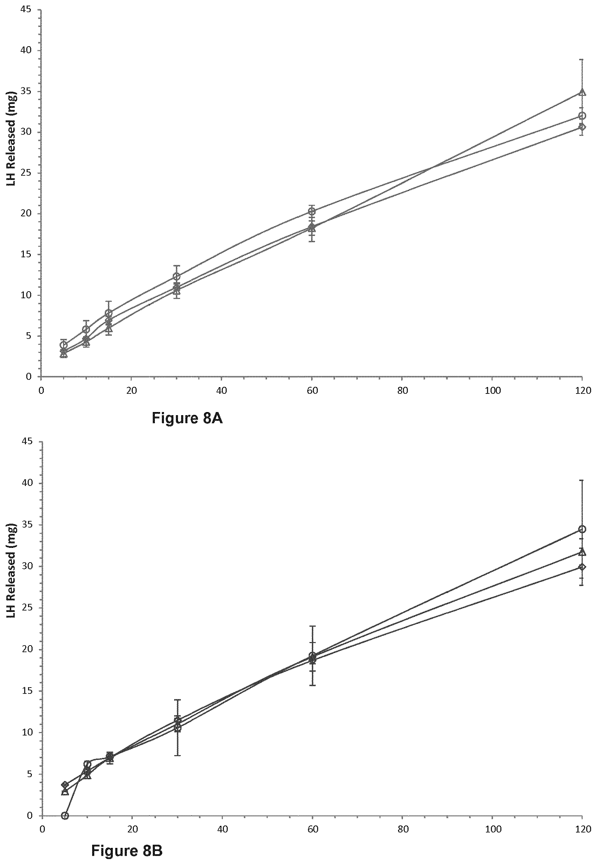

Frequency sweep results for 4-88 stability batches (18 and 19) are presented in Figures 8A and 8B, wherein a solid line with an empty circle represents the IVR for the fresh hydrogel, a solid line with an empty triangle represents the storage (G') moduli for the hydrogel after 3 months and with an empty diamond six months. As can be seen from the Figures, both moduli decrease considerably at all studied stress frequencies for batch numbers 18 (30°C/65 RH) and 19 (20°C uncontrolled RH) over one month storage, remained almost constant during 1-2 month storage and again decreased considerably during 2-3 month storage.

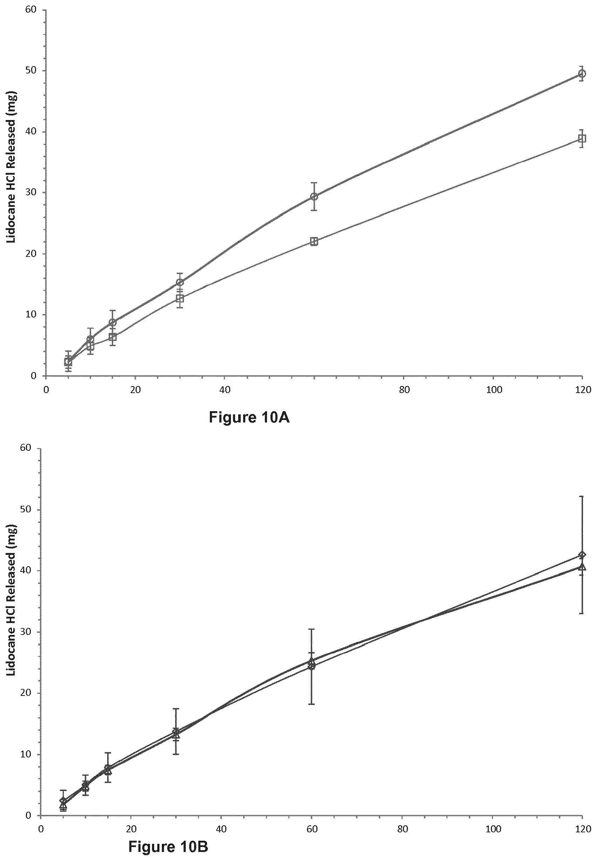

*In vitro* release of lidocaine HCl for batch numbers 18 (30°C/65 RH) and 19 (20°C uncontrolled) after 0 and 3 months storage are graphically depicted in Figures 10A and 10B, respectively. As can be seen from these Figures, the hydrogels displayed the same lidocaine HCl release rate during the first 30 minutes over the 3 month period.

In conclusion, low degree of hydrolysis (88%) PVA blank hydrogels exhibited turbidity, phase separation and an acetic acid-like odour. The occurrence of such changes seemed to be related to the degree of hydrolysis of the PVA chains. No such changes were observed for higher degree of hydrolysis (99%) PVA blank hydrogels. Re-heating experiments confirmed that such changes for 88% hydrolysed PVA hydrogels are not reversed by simple heating and mixing. The effect of heating time while preparing fresh hydrogels, showed that temperature is the main culprit for the stability of the low degree of hydrolysis (88%) PVA hydrogels. Excessive heating at 85°C can cause the fresh 88% hydrolysed PVA hydrogels to de-mix. It was proposed that these changes are attributed to hydrolysis of the residual acetate groups on PVA chains liberating acetic acid decreasing the pH and resulting in phase separation through an unknown mechanism. If the liberated acetic acid can cause degradation of PVA chains, the resultant degraded PVA chains and disturbed ionic interaction may precipitate the less soluble products to generate turbidity.

Lidocaine HCl is stable in both 28-99 and 4-88 PVA-THB hydrogels with no appreciable degradation. Storing gels above 20°C caused a decrease of storage (G') and loss (G") moduli, hardness and compressibility and pH. These observations suggest that the ideal storage condition for the gels is 20°C. Overall stability results for lidocaine HCl loaded PVA-THB hydrogels showed that higher degree of hydrolysis hydrogels are superior than lower degree of hydrolysis PVA hydrogels in almost all physicochemical properties and that lidocaine HCl is generally stable in the formulation at 20°C.

### EXAMPLE 3 - Lidocaine HCl Loaded PVA - THB Hydrogels

Lidocaine loaded gels were manufactured as follows:

| **Material** | **Grade** | **%w/w** | **Quantity (g)** |
|---|---|---|---|
| Lidocaine Hydrochloride monohydrate | Ph. Eur | 4.94 | 4.94 |
| D-Mannitol | Ph. Eur | 2.00 | 2.00 |
| Sodium Tetraborate decahydrate (Borax) | Ph. Eur | 1.80 | 1.80 |
| PVA (99% hydrolysed) (20% stock | Ph. Eur | 12.00 | 60.00 |
| solution | | | |
| De-ionised Water | Not applicable | 79.26 | 31.26 |
| Total | | 100.00 | 100.00 |

Borax, D-mannitol and lidocaine were weighed in individual weigh boats and these powders were added to a pre-weighed 250ml glass jar. De-ionised water was added and the contents of the jar thoroughly mixed. 20% PVA stock solution was added, the lid was closed and the contents of the jar swirled to mix thoroughly before being brought up to final weight using de-ionised water. The jar and its contents were placed in a pre-heated 60°C water bath and the heat turned to 85°C. The mixture was stirred periodically and once homogenous, it was maintained in the 85°C water bath for a further 30 minutes. The suspension was brought up to final weight using de-ionised water and stirred briefly to fully absorb the water before being split into containers (20g of gel per container) for testing.

Autoclaving was conducted using a 200L Front Loading Steam Autoclave by Priorclave, programmed for a USP sterilisation cycle of 121°C for 15 minutes. To ensure the water in the gels did not evaporate, the autoclave was set at 3 bar pressure.

### Method of analysis

Rheometric analysis of 10 gel samples, before and after autoclaving, was carried out using a Kinexus Pro rotational rheometer (Malvern Instruments Ltd., Worcestershire, UK). Storage modulus (G'), loss modulus (G") and complex modulus (G*) were measured following an oscillation stress sweep to determine the linear viscoelastic region. The crossover modulus (where the G' and G" cross) was reported.

### Results

The viscosity and appearance results for the gels (n=10) tested before and after autoclaving is detailed in Table 8 below.

**Table 8: Viscosity and appearance results**

| **Appearance*** | | **Crossover modulus (Pa) (±SD)** | |
|---|---|---|---|
| Before autoclave | After autoclave | Before autoclave* | After autoclave* |
| Clear, homogenous, no air bubbles | Clear, homogenous, no air bubbles | 3.86E+3 (±228.43) | 4.08E+3 (±441.3) |

| | | | |
|---|---|---|---|
| *Average of 10 results | | | |

There was no significant difference between the viscosity of the gels before and after autoclaving. Furthermore, the gels before and after autoclaving were visually equivalent.

### EXAMPLE 4 - Sterilisation results

Lidocaine loaded gels were manufactured as above in both polypropylene and glass containers and according to the following tables:

**Table 9a: Comparative Lidocaine loaded gels**

| **Material** | **Grade** | **%w/w** | **Quantity (g)** |
|---|---|---|---|
| Lidocaine Hydrochloride monohydrate | Ph. Eur | 4.94 | 4.94 |
| Mannitol | Ph. Eur | 2.00 | 2.00 |
| Sodium Tetraborate decahydrate (Borax) | Ph. Eur | 2.50 | 2.50 |
| PVA (87-88% hydrolysed) | Ph. Eur | 16.00 | 16.00 |
| De-ionised Water | Not applicable | 75.26 | Sufficient quantity |
| Total | | 100.00 | 100.00 |

**Table 9b: Lidocaine loaded gels according to the invention**

| **Material** | **Grade** | **%w/w** | **Quantity (g)** |
|---|---|---|---|
| Lidocaine Hydrochloride monohydrate | Ph. Eur | 4.94 | 4.94 |
| Mannitol | Ph. Eur | 2.00 | 2.00 |
| Sodium Tetraborate decahydrate (Borax) | Ph. Eur | 1.80 | 1.80 |
| PVA (99% hydrolysed) | Ph. Eur | 12.00 | 12.00 |
| De-ionised Water | Not applicable | 79.26 | Sufficient quantity |
| Total | | 100.00 | 100.00 |

Autoclaving was conducted using a 200L Front Loading Steam Autoclave by Priorclave, programmed for a USP sterilisation cycle of 121°C for 15 minutes. To ensure the water in the gels did not evaporate, the autoclave was set at 3 bar pressure.

Glass and polypropylene containers are widely used packaging in autoclaves. There was no significant container effect on the stability of the gels after autoclaving.

### Method of analysis

Tackiness was assessed by touch. Prior to sterilisation the gels have no tack. Upon reduction in stability, the gels become sticky and pressing a finger onto the surface and removing it is a simple way to test tackiness. Clarity and air bubbles were assessed visually. All gels prepared were colourless and very transparent, so air bubbles were readily apparent.

Analysis of the shear modulus was carried out using a Kinexus Pro rheometer (Malvern Instruments Ltd., Worcestershire, UK) on all hydrogel formulations. Tests were carried out at 25 °C ± 0.2 °C using 20mm diameter stainless steel parallel plate geometry and a working gap of 1-2 mm. The linear viscoelastic region (LVR) of the PVA-borate hydrogel formulation was determined by an amplitude sweep (0.01-100% strain at a frequency of 1.0 Hz). All measurements of the shear modulus were performed within the LVR.

**Table 10a: Comparative Lidocaine loaded gels - polypropylene container**

| **Measurement taken** | **pH** | **Appearance** | **Tacky** | **Shear modulus (Pa)** |
|---|---|---|---|---|
| immediately before sterilisation | 7.30 | Clear homogenous gel no air bubbles | Yes | 11587 |
| immediately once cooled after 15 minutes heat sterilisation at 121 °C | 7.07 | Clear homogenous gel no air bubbles | Yes | 9974 |
| 8 days after 15 minutes heat sterilisation at 121 °C | 7.10 | Clear homogenous gel no air bubbles | Yes | 9054 |
| 15 days after 15 minutes heat sterilisation at 121 °C | 7.01 | Clear homogenous gel no air bubbles | Yes | 8799 |

| **Measurement taken** | **pH** | **Appearance** | **Tacky** | **Shear modulus (Pa)** |
|---|---|---|---|---|
| immediately before sterilisation | 7.19 | Clear homogenous gel no air bubbles | yes | 12004 |
| immediately once cooled after 15 minutes heat sterilisation at 121 °C | 6.76 | Clear gel | Yes, increased adhesive feel compared to presterilisation | 7987 |
| | | More viscous than before sterilisation | | |
| 8 days after 15 minutes heat sterilisation at 121 °C | 6.88 | Clear gel | Yes, increased adhesive feel compared to presterilisation | 8045 |
| | | Increased adhesive feel | | |
| | | More viscous than before sterilisation | | |
| 15 days after 15 minutes heat sterilisation at 121 °C | 6.85 | Clear gel | Yes, increased adhesive feel compared to presterilisation | 8101 |
| | | Increased adhesive feel | | |
| | | More viscous than before sterilisation | | |

| | | | | |
|---|---|---|---|---|
| Table 10b: Comparative Lidocaine loaded gels - glass container | | | | |

As is clear from Tables 10a and 10b, the shear modulus of lidocaine loaded gels made from 88% hydrolysed PVA decreases upon attempted sterilisation.

**Table 11a: Lidocaine loaded gels according to the invention - polypropylene container**

| **Measurement taken** | **pH** | **Appearance** | **Tacky** | **Shear modulus (Pa)** |
|---|---|---|---|---|
| immediately before sterilisation | 6.78 | Clear homogenous gel no air bubbles | No | 13120 |
| immediately once cooled after 15 minutes heat sterilisation at 121 °C | 6.78 | Clear homogenous gel no air bubbles | No | 13330 |
| 8 days after 15 minutes heat sterilisation at 121 °C | 6.81 | Clear homogenous gel no air bubbles | No | 12960 |
| 15 days after 15 minutes heat sterilisation at 121 °C | 6.74 | Clear homogenous gel no air bubbles | No | 13440 |

**Table 11b: Lidocaine loaded gels according to the invention - glass container**

| **Measurement taken** | **pH** | **Appearance** | **Tacky** | **Shear modulus (Pa)** |
|---|---|---|---|---|
| immediately before sterilisation | 6.90 | Clear homogenous gel no air bubbles | No | 12470 |
| immediately once cooled after 15 minutes heat sterilisation at 121 °C | 6.84 | Clear homogenous gel no air bubbles | No | 12840 |
| 8 days after 15 minutes heat sterilisation at 121 °C | 6.78 | Clear homogenous gel no air bubbles | No | 12780 |
| 15 days after 15 minutes heat sterilisation at 121 °C | 6.91 | Clear homogenous gel no air bubbles | No | 12912 |

As is clear from Tables 11a and 11b, lidocaine loaded gels made from 99% hydrolysed PVA show essentially no difference in shear modulus upon heat sterilisation. Therefore gels according to the invention can be sterilised whilst gels prepared with a lower degree of hydrolysis cannot.

### REFERENCE EXAMPLE 5 - Treatment of Morton's neuroma

Lidocaine loaded gel was prepared as described above in Examples 3 and 4.

A 5g sample of lidocaine loaded gel was placed onto the foot of a patient with an underlying painful Morton's neuroma. The gel was placed onto the top of the foot adjacent the fourth toe. A gauze support (Mepore^{®}) was used to hold the gel in place for 4 hours. After this period of time, the gauze was removed and the patient did not report any sensation of pain from the neuroma.

Aspects of the present invention have been described by way of example only and it should be appreciated that additions and/or modifications may be made thereto without departing from the scope thereof as defined in the appended claims.

## Claims

1. A sterile gel formulation suitable for use in filling a wound cavity and delivering an active ingredient thereto, the gel further having a pH range of 4.5 to 8.5, low bioadhesive strength and cohesive integrity and being formed from a polymer selected from among the group consisting of poly(vinyl) alcohol (PVA) polymer and a PVA-polyvinyl acetate copolymer, a cross-linker being a salt form of boron that produces borate ions in aqueous solution, at least one compound which has a beneficial effect as an active ingredient in the wound and at least one modulator, the modulator being a low molecular weight sugar alcohol that is capable of binding borate or PVA in aqueous solution through a mono-diol or di-diol formation and reduces the pH of PVA-borate hydrogels; wherein the polymer has a degree of hydrolysis of between 98% and 100% and a molecular weight of from 145,000 to 300,000 Daltons, wherein the amount of polymer in the gel formulation ranges from 8 to 20% w/w, and wherein the gel is heat and/or gamma sterilised and contains less than 5% w/w acetic acid.

2. A gel formulation as claimed in Claim 1, wherein the gel formulation is sterilised at 121°C for 15 minutes.

3. A gel formulation as claimed in Claim 1 or Claim 2, wherein the gel contains less than 2% w/w acetic acid, preferably wherein the gel is substantially free of acetic acid.

4. A gel formulation as claimed in any one of Claims 1 to 3, wherein the molecular weight of the polymer is from about 145,000 Daltons to about 200,000 Daltons, preferably about 150,000 Daltons.

5. A gel formulation as claimed in any one of the preceding claims, wherein the amount of borate used in preparing the gel is from about 1.5 to about 3% w/w.

6. A gel formulation as claimed in any one of the preceding claims, wherein the amount of modulator added in the preparation of the gel is from about 0.1 to about 5% w/w.

7. A gel formulation as claimed in any one of the preceding claims, wherein the amount of polymer in the gel formulation ranges from 11 to 15% w/w, preferably 12% w/w.

8. A gel formulation as claimed in any one of the preceding claims, wherein the at least one compound which has a beneficial effect as an active ingredient in the wound is a local anaesthetic, preferably a salt form of lidocaine, particularly preferably lidocaine hydrochloride monohydrate.

9. A gel formulation as claimed in any one of Claims 1 to 7, wherein the at least one compound which has a beneficial effect as an active ingredient in the wound is selected from prilocaine, bupivacaine or another active ingredient that produces a conjugate acid and is stable in the presence of borate ions.

10. A gel formulation as claimed in any one of the preceding claims, wherein the amount of active ingredient added in the preparation of the gel is from 0.1 to 5% w/w.

11. A gel formulation as claimed in any one of the preceding claims for use in wound protection.

12. A gel formulation as claimed in any one of claims 1 to 10 for use in sealing a wound, for use in preventing blood loss from a wound or for use in the removal of debris from a wound, preferably wherein the debris comprises one or more of grit, dust, glass and loose tissue.

13. A gel formulation as claimed in any one of claims 1 to 10 for use in the prevention of wound infection or for use in the treatment of pain.

14. A process for preparing the gel formulation of any one of claims 1 to 10, comprising:
(a) preparing a stock solution of polymer;
(b) preparing a stock solution of cross-linker;
(c) adding the active ingredient and the at least one modulator to the cross-linker solution;
(d) gradually adding the solution prepared in step (c) with stirring to the solution prepared in step (a) to form a gel;
(e) maintaining the resultant gel in a water-bath at 85°C for 30 minutes;
(f) thoroughly stirring the gel to make it as homogenous as possible; and
(g) sterilising the gel with heat or with gamma radiation, preferably wherein heat sterilisation is carried out at 121°C for 15 minutes.

15. A patch comprising the gel formulation of any one of claims 1 to 10 and a porous support.

## Patentansprüche

1. Sterile Gelformulierung, geeignet zur Verwendung beim Füllen einer Wundhöhle und Abgeben eines Wirkstoffs dorthin, wobei das Gel ferner einen pH-Bereich von 4,5 bis 8,5, eine geringe Bioadhäsionsfestigkeit und kohäsive Integrität aufweist und aus einem Polymer gebildet ist, das ausgewählt ist aus der Gruppe bestehend aus Poly(vinyl)alkohol (PVA)-Polymer und einem PVA-Polyvinylacetat-Copolymer, wobei ein Vernetzer eine Salzform von Bor ist, die Borat-Ionen in wässriger Lösung erzeugt, mindestens einer Verbindung, die als Wirkstoff eine günstige Wirkung auf die Wunde aufweist, und mindestens einem Modulator, wobei der Modulator ein niedermolekularer Zuckeralkohol ist, der in der Lage ist, Borat oder PVA in wässriger Lösung durch eine Monodiol- oder Didiolbildung zu binden, und den pH-Wert von PVA-Borat-Hydrogelen senkt; wobei das Polymer einen Hydrolysegrad zwischen 98 % und 100 % und eine Molekülmasse von 145.000 bis 300.000 Dalton aufweist, wobei die Menge des Polymers in der Gelformulierung im Bereich von 8 bis 20 % w/w liegt und wobei das Gel hitze- und/oder gammasterilisiert ist und weniger als 5 % w/w Essigsäure enthält.

2. Gelformulierung nach Anspruch 1, wobei die Gelformulierung 15 Minuten lang bei 121 °C sterilisiert wird.

3. Gelformulierung nach Anspruch 1 oder Anspruch 2, wobei das Gel weniger als 2 % w/w Essigsäure enthält, wobei vorzugsweise das Gel im Wesentlichen frei von Essigsäure ist.

4. Gelformulierung nach einem der Ansprüche 1 bis 3, wobei die Molekülmasse des Polymers etwa 145.000 Dalton bis etwa 200.000 Dalton, vorzugsweise etwa 150.000 Dalton, beträgt.

5. Gelformulierung nach einem der vorhergehenden Ansprüche, wobei die beim Herstellen des Gels verwendete Menge von Borat etwa 1,5 bis etwa 3 % w/w beträgt.

6. Gelformulierung nach einem der vorhergehenden Ansprüche, wobei die Menge des bei der Herstellung des Gels zugegebenen Modulators etwa 0,1 bis etwa 5 % w/w beträgt.

7. Gelformulierung nach einem der vorhergehenden Ansprüche, wobei die Menge von Polymer in der Gelformulierung im Bereich von 11 bis 15 % w/w, vorzugsweise 12 % w/w liegt.

8. Gelformulierung nach einem der vorhergehenden Ansprüche, wobei die mindestens eine Verbindung, die als Wirkstoff eine günstige Wirkung auf die Wunde aufweist, ein Lokalanästhetikum ist, vorzugsweise eine Salzform von Lidocain, besonders bevorzugt Lidocainhydrochloridmonohydrat.

9. Gelformulierung nach einem der Ansprüche 1 bis 7, wobei die mindestens eine Verbindung, die als Wirkstoff eine günstige Wirkung auf die Wunde aufweist, aus Prilocain, Bupivacain oder einem anderen Wirkstoff ausgewählt ist, der eine konjugierte Säure erzeugt und in Gegenwart von Borat-Ionen stabil ist.

10. Gelformulierung nach einem der vorhergehenden Ansprüche, wobei die Menge des bei der Herstellung des Gels zugesetzten Wirkstoffs 0,1 bis 5 % w/w beträgt.

11. Gelformulierung nach einem der vorhergehenden Ansprüche zur Verwendung beim Wundschutz.

12. Gelformulierung nach einem der Ansprüche 1 bis 10 zur Verwendung beim Verschließen einer Wunde, zur Verwendung zum Verhindern von Blutverlust aus einer Wunde oder zur Verwendung zum Entfernen von Fremdkörpern aus der Wunde, wobei vorzugsweise die Fremdkörper eines oder mehrere von Sand, Staub, Glas und loses Gewebe umfassen.

13. Gelformulierung nach einem der Ansprüche 1 bis 10 zur Verwendung bei der Vorbeugung von Wundinfektionen oder zur Verwendung bei der Behandlung von Schmerzen.

14. Verfahren zum Herstellen der Gelformulierung nach einem der Ansprüche 1 bis 10, umfassend:
(a) Herstellen einer Polymer-Stammlösung;
(b) Herstellen einer Vernetzer-Stammlösung;
(c) Zugeben des Wirkstoffs und des mindestens einen Modulators zur Vernetzer-Lösung;
(d) allmähliches Zugeben der in Schritt (c) hergestellten Lösung unter Rühren zu der in Schritt (a) hergestellten Lösung, um ein Gel zu bilden;
(c) Halten des resultierenden Gels 30 Minuten lang in einem Wasserbad bei 85 °C;
(f) gründliches Rühren des Gels, um es so homogen wie möglich zu machen; und
(g) Sterilisieren des Gels durch Hitze oder durch Gammastrahlung, wobei vorzugsweise die Hitzesterilisation 15 Minuten lang bei 121 °C durchgeführt wird.

15. Pflaster, das die Gelformulierung nach einem der Ansprüche 1 bis 10 und einen porösen Träger umfasst.

## Revendications

1. Formulation de gel stérile appropriée pour être utilisée dans le remplissage d'une cavité de plaie et l'administration d'un principe actif dans celle-ci, le gel comportant en outre une plage de pH de 4,5 à 8,5, une faible résistance bioadhésive et une intégrité cohésive et étant formée à partir d'un polymère choisi dans le groupe constitué par un polymère d'alcool polyvinylique (PVA) et un copolymère PVA-acétate de polyvinyle, un agent de réticulation étant une forme de sel de bore qui produit des ions borate en solution aqueuse, au moins un composé qui comporte un effet bénéfique en tant que principe actif dans la plaie et au moins un modulateur, le modulateur étant un alcool de sucre de faible poids moléculaire qui est capable de se lier au borate ou au PVA en solution aqueuse par l'intermédiaire de la formation d'un mono-diol ou d'un di-diol et réduit le pH des hydrogels PVA-borate ; ledit polymère comportant un degré d'hydrolyse entre 98 % et 100 % et un poids moléculaire entre 145 000 et 300 000 Daltons, ladite quantité de polymère dans la formulation de gel étant comprise entre 8 et 20 % en p/p et ledit gel étant chauffé et/ou stérilisé aux rayons gamma et contenant moins de 5 % en p/p d'acide acétique.

2. Formulation de gel selon la revendication 1, ladite formulation de gel étant stérilisée à 121°C pendant 15 minutes.

3. Formulation de gel selon la revendication 1 ou la revendication 2, ledit gel contenant moins de 2 % en p/p d'acide acétique, de préférence ledit gel étant sensiblement exempt d'acide acétique.

4. Formulation de gel selon l'une quelconque des revendications 1 à 3, ledit poids moléculaire du polymère étant compris entre environ 145 000 Daltons et environ 200 000 Daltons, de préférence d'environ 150 000 Daltons.

5. Formulation de gel selon l'une quelconque des revendications précédentes, ladite quantité de borate utilisée dans la préparation du gel étant comprise entre environ 1,5 et environ 3 % en p/p.

6. Formulation de gel selon l'une quelconque des revendications précédentes, ladite quantité de modulateur ajoutée dans la préparation du gel étant comprise entre environ 0,1 et environ 5 % en p/p.

7. Formulation de gel selon l'une quelconque des revendications précédentes, ladite quantité de polymère dans la formulation de gel étant comprise entre 11 à 15 % en p/p, de préférence de 12 % en p/p.

8. Formulation de gel selon l'une quelconque des revendications précédentes, ledit au moins un composé qui comporte un effet bénéfique en tant que principe actif dans la plaie étant un anesthésiant local, de préférence une forme de sel de lidocaïne, en particulier de préférence le chlorhydrate de lidocaïne monohydraté.

9. Formulation de gel selon l'une quelconque des revendications 1 à 7, ledit au moins un composé qui comporte un effet bénéfique en tant que principe actif dans la plaie étant choisi parmi la prilocaïne, la bupivacaïne ou un autre principe actif qui produit un acide conjugué et est stable en présence d'ions borate.

10. Formulation de gel selon l'une quelconque des revendications précédentes, ladite quantité de principe actif ajoutée dans la préparation du gel étant comprise entre 0,1 à 5 % en p/p.

11. Formulation de gel selon l'une quelconque des revendications précédentes destinée à être utilisée dans la protection de plaies.

12. Formulation de gel selon l'une quelconque des revendications 1 à 10 destinée à être utilisée dans la fermeture d'une plaie, destinée à être utilisée dans la prévention des pertes de sang d'une plaie ou destinée à être utilisée dans la suppression des débris d'une plaie, de préférence lesdits débris comprenant un ou plusieurs éléments parmi du gravier, de la poussière, du verre et un tissu lâche.

13. Formulation de gel selon l'une quelconque des revendications 1 à 10 destinée à être utilisée dans la prévention de l'infection de plaies ou destinée à être utilisée dans le traitement de la douleur.

14. Procédé de préparation de la formulation de gel selon l'une quelconque des revendications 1 à 10, comprenant :
(a) la préparation d'une solution-mère de polymère ;
(b) la préparation d'une solution-mère d'agent de réticulation ;
(c) l'ajout du principe actif et dudit au moins un modulateur à la solution d'agent de réticulation ;
(d) l'ajout progressif de la solution préparée à l'étape (c) sous agitation à la solution préparée à l'étape (a) pour former un gel ;
(c) le maintien du gel résultant dans un bain-marie à 85°C pendant 30 minutes ;
(f) l'agitation minutieuse du gel pour le rendre aussi homogène que possible ; et
(g) la stérilisation du gel par la chaleur ou par rayonnement gamma, de préférence ladite stérilisation par la chaleur étant effectuée à 121°C pendant 15 minutes.

15. Timbre dermique comprenant la formulation de gel selon l'une quelconque des revendications 1 à 10 et un support poreux.
